# EUROPÄISCHE PATENTSCHRIFT

(11) **EP 3 432 057 B1**
(45) Veröffentlichungstag und Bekanntmachung des Hinweises auf die Patenterteilung: **09.12.2026**
(21) Anmeldenummer: 18188534.4
(22) Anmeldetag: 08.05.2010
(51) Int. Cl.: G02C 13/00, A61B 5/11

(54) **METHODE UND VORRICHTUNG ZUR ERMITTLUNG DER HABITUELLEN KOPFHALTUNG**
METHOD AND UNIT FOR DETERMINING HABITUAL HEAD POSTURE
PROCÉDÉ ET DISPOSITIF DE DÉTERMINATION DU PORT DE TÊTE HABITUEL

(30) Priorität: 17.06.2009 DE 102009025215
(43) Veröffentlichungstag der Anmeldung: 23.01.2019
(60) Teilanmeldung: 21157838.0 / 3 842 855
(62) Dokumentnummer(n) der früheren Anmeldung(en) nach Art. 76 EPÜ: 10722933.8 / 2 467 751
(73) Patentinhaber: Carl Zeiss Vision International GmbH, 73430 Aalen (DE)
(72) Erfinder: Kubitza, Matthias, 73434 Aalen (DE); Gamperling, Michael, 89340 Leipheim (DE); Cabeza-Guillén, Jesús-Miguel, 73434 Aalen (DE)
(74) Vertreter: GLAWE DELFS MOLL

(56) Entgegenhaltungen:
- EP-A1- 1 637 067
- EP-A2- 0 898 930
- WO-A1-2006/029875
- WO-A2-2007/045694
- CN-A- 101 378 693
- DE-A1- 102004 063 160
- DE-A1- 102008 018 198
- DE-U1- 29 910 680
- FR-A1- 2 896 682
- KR-A- 20080 094 079
- US-A1- 2003 143 391
- US-A1- 2009 207 375
- MICHAEL OSWALD ET AL: "Visioffice und eyecode: Die Messung für perfektes Sehen - Teil 1 Visioffice", DEUTSCHE OPTIKER ZEITUNG DOZ, no. 5-2009, 1 January 2009 (2009-01-01), pages 46 - 50, XP055005882

## Beschreibung

**Die** Erfindung betrifft ein Verfahren zur Zentrierdatenermittlung nach dem Oberbegriff des Patentanspruchs 1 und eine Vorrichtung zur Zentrierdatenermittlung nach dem Oberbegriff des Patentanspruchs 8.

Schließlich betrifft die Erfindung ein Computerprogramm zur Durchführung des vorstehend angegebenen Verfahrens.

Wenn Augenoptiker zur optischen Anpassung einer Brille die Zentrierdaten, also alle notwendigen Angaben zur korrekten Zentrierung von Brillengläsern in einer Brillenfassung für den individuellen Anwendungszweck des Kunden oder Probanden, ermittelt, so kann wie folgt verfahren werden:
Der Kunde wird gebeten eine möglichst natürliche Kopf- und Körperhaltung einzunehmen. Die Festlegung der Zentrierwerte erfolgt dann durch Markierung der Durchblickpunkte in der Scheibenebene der Brillenfassung mittels Stift oder mittels Videoaufnahme und anschließender manueller (wie z.B. bei der von der Anmelderin unter der Marke "Video Infral" vertriebenen Videozentriereinrichtung) bzw. automatisierter (wie z.B. bei der von der Anmelderin unter der Marke "RV Terminal" vertriebenen Videozentriereinrichtung) Zentrierdatenermittlung.

Zur Ermittlung des Vorneigungswinkels ist ein Hilfsmittel Namens "Y-Stick" bekannt, welches in einem Zeitpunkt den Vorneigungswinkel der Brillenfassung "einfriert", damit dieser anschließend abgelesen werden kann. Der so erfasste Vorneigungswinkel wird in einem nachfolgenden Schritt dazu benutzt, den Kunden in gleicher Position vor einem Zentriersystem zu positionieren.

Weiterhin ist aus der EP 1 591 064 A1 ein Messgerät zur Bestimmung der Kopfneigung bekannt, welches von der Anmelderin der EP 1 591 064 unter der Bezeichnung "Ysis" vertrieben wird. Dieses Messgerät wird an der Brillenfassung befestigt. Dieser Vorschlag zielt darauf ab, die "normale" Kopfhaltung zu einem Zeitpunkt jeweils für Nah- und Fernblickrichtung festzuhalten. Aus den Kopfneigungen, die beim Blick in die Ferne sowie beim Lesen eingenommen werden, wird der Unterschied der Kopfneigung zwischen Fern- und Nahsehaufgaben ermittelt. Daraus werden dann die zugehörigen Durchblickpunkte durch das Brillenglas bestimmt.

Sämtliche vorstehenden Methoden sind statisch, d.h. zur Ermittlung einer habituellen Kopfhaltung wird eine "Momentaufnahme" der jeweiligen Situation herangezogen.

Die WO 01/62139 A1 offenbart, das Verhalten eines Brillenträgers hinsichtlich seiner Blickgewohnheiten zu klassifizieren. Es wird die typische Kopf- und Augenbewegung bei verschiedenen Sehaufgaben bestimmt. Davon abhängig, wird das Blickverhalten des Probanden kategorisiert nach "Beweglichkeit" der Augen und des Kopfes ("head/eye mover"). Die Ergebnisse der Analyse werden dabei als Grundlage für die Auswahl eines geeigneten Brillenglases verwendet.

Aus der EP 1 747 750 A1 ist es bekannt, die häufigsten Blickrichtungen relativ zum Kopf festzustellen. Zu diesem Zweck werden Kopfhaltung und Blickrichtung gleichzeitig detektiert. Aus den ermittelten relativen Blickrichtungen wird die relative "Netto"-Blickrichtung und der daraus resultierende Durchblickpunkt durch ein Brillenglas bestimmt.

Die DE 2004 063 160 A1 beschreibt ein Verfahren und eine Einrichtung zum Anpassen einer Brille gemäß des Oberbegriffs der Patentansprüche 1 und 8, insbesondere zur Erfassung der Habitus-Kopfhaltung eines Probanden, dessen bzw. deren Messdaten Eingang finden bei der Video-Zentrierdaten-Bestimmung. Bei dem Verfahren werden vom Probanden mehrere Video-Fotos zur Ermittlung der Kopfhaltungswinkel eines Probanden in verschiedenen Kopfhaltungspositionen gefertigt, die Kopfhaltungswinkel erfasst und mit dem Vorneigungswinkel der Brillenfassung sowie dem Hornhautscheitelabstand abgeglichen. Die entsprechenden Video-Fotos werden in einem Moment aufgenommen, wenn nach Meinung des Bedienenden der Proband seine natürliche Habitus-Kopfhaltung eingenommen hat.

Eine Zentriermessung, also eine Messung, bei der alle notwendigen Angaben zur korrekten Zentrierung von Brillengläsern in einer Brillenfassung für den individuellen Anwendungszweck des Kunden oder Probanden, ermittelt werden, stellt für den Kunden des Optikers eine erzwungene Situation dar. Zwar ist aus der Zentriermessung bekannt, wie der Kunde während einer Aufnahme den Kopf hielt, diese Kopfhaltung entspricht allerdings nicht zwangsläufig seiner "normalen", d.h. häufigsten Kopfhaltung in tatsächlich ungezwungenen Situationen.

Dies ändert sich auch nicht, wenn das in der DE 10 2004 063 160 A1 beschriebene Verfahren zum Einsatz kommt. Darüber hinaus birgt dieses Verfahren die Gefahr, dass gar nicht die vermeintlich natürliche Kopfhaltung aufgenommen wird, sonder eine andere, weil der Proband innerhalb der Reaktionszeit der Bedienenden bereits eine andere Kopfhaltung eingenommen hat. Die Aufgabe der Erfindung besteht nunmehr darin, ein verbessertes Verfahren zur Ermittlung der habituellen Kopfhaltung eines Probanden bereitzustellen, und zwar dergestalt, dass die ermittelte Kopfhaltung der tatsächlichen "normalen" Kopfhaltung des Probanden näher kommt, als dies bei den oben beschriebenen Verfahren zum Stand der Technik der Fall ist. Eine weitere Aufgabe der Erfindung besteht darin, eine entsprechende Vorrichtung zur präziseren Ermittlung der habituellen Kopfhaltung eines Probanden bereitzustellen.

Die erstgenannte Aufgabe wird durch ein gattungsgemäßes Verfahren zur Ermittlung der habituellen Kopfhaltung eines Probanden mit den Merkmalen des kennzeichnenden Teils des Patentanspruchs 1 gelöst.

Die danach angegebene Aufgabe wird durch eine gattungsgemäße Vorrichtung zur Ermittlung der habituellen Kopfhaltung eines Probanden mit den Merkmalen des kennzeichnenden Teils des Patentanspruchs 8 gelöst.

Vorteilhafte Ausführungen und Weiterbildungen der Erfindung sind Gegenstand der Unteransprüche.

Ein nicht von den Ansprüchen umfasstes Verfahren zur Ermittlung der habituellen Kopfhaltung eines Probanden umfasst die folgenden Verfahrensschritte:
a. Aufzeichnung der Kopfhaltung über eine gewisse Zeitspanne und
b. Ermittlung einer bevorzugten Kopfhaltung aus der aufgezeichneten Kopfhaltung.

Die entsprechende Vorrichtung zur Ermittlung der habituellen Kopfhaltung des Probanden umfasst in korrespondierender Weise:
a. eine Aufzeichnungseinrichtung zur Aufzeichnung der Kopfhaltung über die Zeitspanne und
b. eine Ermittlungseinrichtung zur Ermittlung der bevorzugten Kopfhaltung aus der aufgezeichneten Kopfhaltung.

Durch das vorstehend skizzierte Verfahren bzw. die insbesondere zur Durchführung dieses Verfahrens ausgebildete entsprechende Vorrichtung wird vermieden, dass die habituelle Kopfhaltung des Probanden irrtümlich als eine zufällig zu einem Zeitpunkt aufgenommene bzw. bestimmte Kopfhaltung angenommen wird, die gerade nicht seiner gewöhnlichen Kopfhaltung entspricht, wie dies nach den in der Beschreibungseinleitung beschriebenen Verfahren aus dem Stand der Technik der Fall sein kann. Die eingangs gestellte Aufgabe wird durch dieses Verfahren bzw. die entsprechende Vorrichtung also vollumfänglich gelöst.

Die Annahme der habituellen Kopfhaltung als die aus einer über eine gewisse Zeit hinweg aufgezeichneten Kopfhaltung ermittelte bevorzugte Kopfhaltung ermöglicht es z.B. auch, die bei der Zentriermessung (z.B. mit Hilfe der unter den Bezeichnungen Relaxed Vision Terminal und Video Infral von der Anmelderin vertriebenen Videozentriergeräten) gemessene Kopfhaltung zu korrigieren, so dass die Zentrierdaten auf Basis der "normalen" und nicht der zufälligen Kopfhaltung bestimmt werden.

Die Erfindung umfasst auch ein Verfahren zur Zentrierdatenermittlung nach Anspruch 1, bei dem unter anderen
a. eine tatsächliche momentane Kopfhaltung eines Probanden bestimmt wird,
b. eine Anordnung einer Brillenfassung relativ zur bestimmten tatsächlichen momentanen Kopfhaltung ermittelt wird,
c. eine mit einem automatisierten Verfahren habituelle Kopfhaltung bestimmt wird und
d. die bestimmte tatsächliche momentane Kopfhaltung mit Hilfe der automatisiert bestimmten habituellen Kopfhaltung korrigiert wird.

In entsprechender Weise ist Gegenstand der Erfindung auch eine Vorrichtung zur Zentrierdatenermittlung nach Anspruch 8, unter anderem mit
a. einer Kopfhaltungsbestimmungseinrichtung, um die tatsächliche momentane Kopfhaltung des Probanden zu bestimmen,
b. einer Ermittlungseinrichtung, um die Anordnung der Brillenfassung relativ zur bestimmten tatsächlichen momentanen Kopfhaltung zu ermitteln,
c. einer Vorrichtung zur automatisierten Bestimmung der habituellen Kopfhaltung und
d. einer Korrektureinrichtung zur Korrektur der bestimmten tatsächlichen momentanen Kopfhaltung mit Hilfe der bestimmten habituellen Kopfhaltung.

Bei dem Verfahren zur Ermittlung der habituellen Kopfhaltung eines Probanden kann die Zeitspanne, innerhalb der die Kopfhaltung aufgezeichnet wird, vorgegeben werden. So kann zum Beispiel die Kopfhaltung nur während der Zeit aufgezeichnet werden, während der sich auch verlässliche Daten zur habituellen Haltung des Kopfes des Probanden entnehmen lassen. Die Aufzeichnung erzwungener und/oder unnatürlicher Kopfhaltungen kann vermieden werden. Darüber hinaus lässt sich die Zeitspanne individuell so festlegen, dass das aufgenommene Datenmaterial eine hinreichend verlässliche Aussage über die tatsächliche habituelle Kopfhaltung zulässt. Günstig ist es z.B., wenn aus der Aufzeichnung nur solche Zeitabschnitte zur Ermittlung der habituellen Kopfhaltung ausgewählt werden, innerhalb derer der Proband auch eine Kopf- und/oder Körperhaltung eingenommen hat, die ergonomischen Grundsätzen entspricht.

Als bevorzugte Kopfhaltung ist die Kopfhaltung zu verstehen, in der der Proband seinen Kopf bei einer vorgegebenen Sehaufgabe mit einer gewissen Wahrscheinlichkeit hält. Typische Werte für diese Wahrscheinlichkeit sind z.B. über 30 %, über 50 %, über 80 % oder gar über 90 % für die bestimmte Sehaufgabe. Eine derartige Analyse lässt sich mit üblichen mathematischen/statistischen Methoden durchführen. Geeignete Software Algorithmen können bei Bedarf automatisch oder mit Unterstützung durch den Benutzer Extremwerte (Ausreißer) aus der aufgezeichneten Datenmenge der habituellen Kopfhaltung eliminieren, bevor eine Bestimmung einer typischen Kopfhaltung erfolgt. Es erfolgt dann z.B. ein Auswahl aus der Menge dieser wahrscheinlichsten Kopfhaltungen.

Als bevorzugte Kopfhaltung kann auch die aufgezeichnete Haltung des Kopfes gewählt werden, in der der Proband den Kopf mit der größten Wahrscheinlichkeit hält.

Obwohl es grundsätzlich möglich ist, dem Probanden freizustellen, in welcher Körperhaltung und/oder was er bei der Aufzeichnung seiner Kopfhaltung betrachtet, ist es vielfach günstig, diesem bestimmte Sehaufgaben aufzuerlegen. Insbesondere wenn die Kopfhaltung Eingang in die Brillenglasberechnung oder die Zentrierung eines Brillenglases findet, ist es sinnvoll, besondere Aufmerksamkeit den Blickrichtungen für die Nähe und/oder die Ferne zu widmen. Die Erfindung sieht z.B. vor, als bevorzugte Kopfhaltung eine der aufgezeichneten Haltungen zu wählen, in der der Proband den Kopf beim Blick in die Nähe und/oder beim Blick in die Ferne hält.

Weiterhin ist es sinnvoll, die Körperhaltung des Probanden zu beachten. Beim Blick in die Ferne wird eine Person eher stehen, wohingegen eine Person beim Blick in die Nähe, wie z.B. beim Lesen, bevorzugt eine sitzende Position einnimmt. Die Erfindung sieht daher vor, als bevorzugte Kopfhaltung eine der aufgezeichneten Haltungen zu wählen, während der der Proband steht und/oder sitzt.

Die habituelle Kopfhaltung hängt in besonderem Maß von der Tätigkeit des Probanden und den visuellen Eindrücken, die der Proband wahrnimmt ab. Es ist daher weiter günstig, dem Probanden konkrete Alltagssituationen und/oder alltägliche Sehaufgaben darzubieten unter denen seine habituelle Kopfhaltung bestimmt werden soll. Der hiermit verbundene Vorteil liegt darin, dass der Proband/Kunde von der eigentlichen Messsituation abgelenkt ist und damit keine unnatürliche Kopfhaltung einnimmt. Die Sehaufgaben können darin bestehen, dass der Proband/Kunde sich z.B. im Laden des Optikers, der die habituelle Kopfhaltung aufzeichnet frei bewegt und z.B. die Auslagen betrachtet oder dass er z.B. Verkehrssituationen verfolgt, die über ein elektronisches Medium, wie ein Display, einen Beamer usw., dargestellt werden, wie dies z.B. in der US 2007/0229761A1 beschrieben ist oder dass er gebeten wird ein Schriftstück durchzulesen. Die Sehaufgaben werden z.B. so gewählt, dass eine typische Kopfhaltung im Stehen und/oder im Sitzen bestimmt werden kann. Bei der Zentrierdatenermittlung für eine Lesebrille wird z.B. die habituelle Kopfhaltung zu berücksichtigen sein, während der der Proband sitzt und liest.

Konkret können dem Probanden/Kunden zur dynamischen Ermittlung der habituellen Kopfhaltung über ein Zeitintervall Alltagssituationen bzw. alltägliche Sehaufgaben im Nah- und/oder Fernbereich dargeboten werden. In dieser Zeit werden die Kopfhaltung, ggf. in Kombination mit der zugehörigen Körperhaltung und ggf. auch die (relative) Position wie z.B. die Neigung der Brille bzw. der Brillengläser, die der Proband trägt, fortlaufend gemessen. Die Messung erfolgt so, dass der Proband/Kunde davon nicht beeinträchtigt wird. Im Anschluss an die Messung kann die jeweils häufigste Kopfhaltung, soweit mit aufgezeichnet auch die zugehörige Körperhaltung- und ggf. auch die Position, insbesondere Neigung der Brille bzw. der Brillengläser bestimmt und später bei der Ermittlung der Zentrierdaten berücksichtigt werden. Insbesondere können die Durchblickpunkte bei Nah- und/oder Fernsehaufgaben, der sogenannte Inset und/oder die Korridorlänge bestimmt werden. Konkret kann z.B. aus der Kopfhaltung beim Sehen im Nah- und Fernbereich bei bekannter Position des Sehziels der Durchblickpunkt durch das Brillenglas bestimmt werden. Sind die Durchblickpunkte bekannt, kann damit auch die Größe des Insets bestimmt werden, sowie die Länge des Korridors des Nahteils. Ein eventueller Unterschied zwischen Kopfrichtung und Blickrichtung kann bei der Bestimmung der Durchblickpunkte und/oder des Insets mit berücksichtigt werden.

Die Kopfhaltung des Probanden kann auf unterschiedlichste Art und Weise ermittelt werden. So kann erfindungsgemäß z.B. die Aufzeichnung der Kopfhaltung mittels eines am Kopf des Probanden angebrachten Positionsaufzeichnungsgeräts durchgeführt werden. Dieses Positionsaufzeichnungsgerät kann mittelbar also z.B. am Brillenfassungsbügel oder unmittelbar am Kopf des Klienten befestigt sein. Dieses Positionsaufzeichnungsgerät kann nach dem Prinzip eines Datenloggers mit anschließendem Datentransfer über eine Basisstation funktionieren oder der Datentransfer kann zeitgleich über eine drahtlose Verbindung, wie WPAN (Wireless Personal Area Network), nämlich z.B. via Bluetooth, oder WLAN (Wireless Local Area Network) online während des gesamten Messintervalls erfolgen. Das Positionsaufzeichnungsgerät kann entweder absolute Lageinformationen messen oder aber Änderungen in der Lage. Im zweiten Fall muss der Messwertaufnehmer vor und/oder nach dem Messvorgang in eine Referenzlage gebracht werden, deren Ausrichtung bekannt ist. Als Referenzlage kann z.B. eine horizontale Tischfläche oder die Auflage einer speziell dafür vorgesehenen Halterung dienen.

Die Aufzeichnung der Kopfhaltung kann erfindungsgemäß zusätzlich oder alternativ mittels eines Bildaufzeichnungsgeräts durchgeführt werden. Ein derartiges Bildaufzeichnungsgerät kann z.B. ein Video- oder Digitalkamerasystem mit angeschlossener automatisierter Bildauswertung sein. Die Bilddaten können entweder als zweidimensionale (2D) Daten direkt oder nach Erstellung eines dreidimensionalen (3D) Modells als 3D-Daten weiterverarbeitet werden. Aus diesen Daten können z.B. auch typische Parameter wie Vorneigung, Seitenneigung, usw., der Brille bestimmt werden.

Bei dem erfindungsgemäßen Verfahren zur Zentrierdatenermittlung wird die bestimmte tatsächliche momentane Kopfhaltung mit Hilfe der bestimmten habituellen Kopfhaltung derart korrigiert, dass die Zentrierdaten auf Basis der habituellen und nicht der zufälligen Kopfhaltung bestimmt werden. Die Korrektur kann zum Beispiel erfolgen, indem die Abweichung der momentanen Kopfhaltung von der habituellen Kopfhaltung ermittelt wird und anhand dieser Abweichung eine Umrechnung/Korrektur der Zentrierwerte erfolgt.

Die ermittelte habituelle Kopfhaltung des Probanden/Kunden und der ggf. ebenfalls ermittelte Vorneigungswinkel der Brille können auch zur erzwungenen Ausrichtung des Kunden für die konventionelle Zentrierdatenermittlung dienen, indem bei Erreichen des angestrebten Vorneigungswinkels der Brille eine automatische Auslösung der bildgebenden Einheiten eingeleitet wird bzw. ein Signal dem Operator gegeben wird, dass nun die Zentrierdatenermittlung erfolgen kann.

Die Erfindung sieht insbesondere auch vor, dass die vorstehend beschriebenen Verfahren zur Zentrierdatenermittlung in Form von in einem Computer ausführbaren Computerprogrammen mit Programmcodes vorliegen. Die Computerprogramme können auf einem maschinenlesbaren Datenträger gespeichert sein.

Der Vollständigkeit halber wird darauf hingewiesen, dass die o.a. Aufzeichnungseinrichtung zur Aufzeichnung der Kopfhaltung über eine Zeitspanne ein am Kopf des Probanden anbringbares Positionsaufzeichnungsgerät und/oder ein Bildaufzeichnungsgerät umfassen kann. Positionsaufzeichnungsgerät und/oder Bildaufzeichnungsgerät können z.B. in der vorstehend beschriebenen Weise ausgebildet sein. Die Ermittlungseinrichtung zur Ermittlung einer bevorzugten Kopfhaltung aus der Aufzeichnung kann einen Computer umfassen.

Die Kopfhaltungsbestimmungseinrichtung zur Bestimmung der tatsächlichen momentanen Kopfhaltung eines Probanden kann ein Positionsaufzeichnungsgerät und/oder ein Bildaufzeichnungsgerät, insbesondere der vorstehend beschriebenen Art, umfassen.

Die Ermittlungseinrichtung zur Ermittlung der Anordnung einer Brillenfassung relativ zur bestimmten tatsächlichen momentanen Kopfhaltung kann ein Bildaufzeichnungsgerät, insbesondere eine Video- oder Digitalkamera, und/oder einen Computer umfassen.

Die Erfindung wird nunmehr anhand der Zeichnung näher beschrieben. Es zeigen:
- Figur 1: eine schematische Darstellung einer erfindungsgemäßen Vorrichtung zur Zentrierdatenermittlung mit erfindungsgemäßer Vorrichtung zur Ermittlung der habituellen Kopfhaltung eines Probanden;
- Figur 2: Aufnahmen des Gesichts eines Kunden mit angepasster Fassung in Nullblickrichtung
a. von vorn
b. von der Seite
c. von vorn mit Zentrierdaten, nämlich x, y
d. von der Seite mit Zentrierdaten, nämlich Hornhautscheitelabstand und Fassungsvorneigung

Es wird angenommen, dass ein Kunde ein Optikergeschäft zum Zwecke des Erwerbs einer neuen Brille aufsucht. Zunächst wird er hinsichtlich der für ihn in Frage kommenden Brillengläser und Fassungen beraten. Nach erfolgter Auswahl der Fassung erfolgt die eigentliche Zentrierdatenermittlung mit Hilfe einer erfindungsgemäßen Vorrichtung.

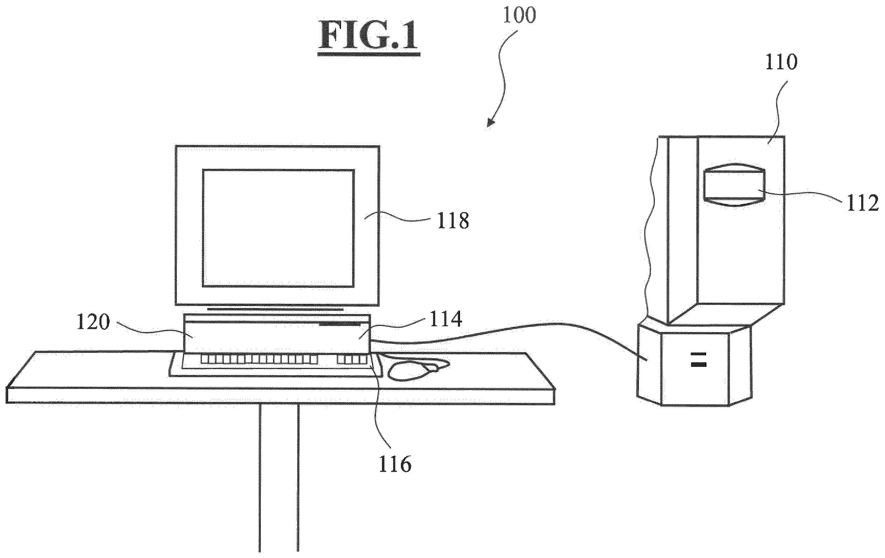

Die Figur 1 zeigt eine erfindungsgemäße Vorrichtung zur Zentrierdatenermittlung 100. Diese Vorrichtung zur Zentrierdatenermittlung 100 umfasst ein Videozentriergerät 110 mit Videokamera 112 und Computer 114 mit Tastatur 116, Bildschirm 118 und Speichermedium 120. Es handelt sich um ein kalibriertes System, also ein System zur Aufnahme und Bestimmung von Abmessungen.

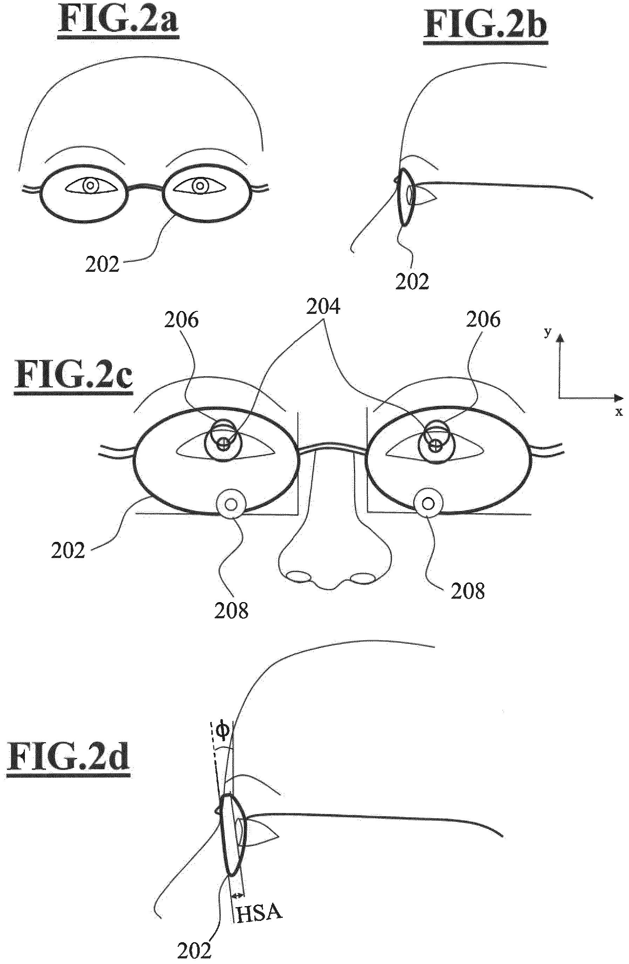

Das Gesicht des Kunden wird mit angepasster Fassung 202 in Nullblickrichtung von vom und von der Seite mit der Videokamera 112 aufgenommen, wie dies in den Figuren 2a) und b) dargestellt ist.

Mit der auf den Bildschirm 118 dargestellten Frontaufnahme (Figur 2c) wird zunächst die Fassungsform 202 erfasst. Weiterhin werden die Pupillenmitten 204, z.B. im Koinzidenzverfahren, relativ zur Fassungsform 202 ermittelt. Mit der Seitenaufnahme werden der Hornhautscheitelabstand HSA und die Fassungsvorneigung φ zur Erfüllung der Augendrehpunktforderung ermittelt, wie dies in Figur 2d) dargestellt ist.

Erfindungsgemäß ist nunmehr vorgesehen, die habituelle Kopfhaltung und die Neigung der Brille z.B. mit Hilfe der kalibrierten Videokamera über einen längeren Zeitraum aufzuzeichnen und hieraus eine habituelle Kopfhaltung und eine bevorzugte Neigung der Brille zu ermitteln.

Die somit ermittelte Kopfhaltung und der damit zusammenhängende Vorneigungswinkel der Brille sowie andere Messdaten wie z.B. die Anordnung der Brille in der Fassungs- oder Scheibenebene in x, y dienen zur Umrechnung der mittels des Videozentriergeräts 110 konventionell ermittelten Zentrierdaten (z.B. Fernbezugspunkt 206, Nahmesskreis 208), falls bei deren Bestimmung der Kunde eine von der normalen Kopfhaltung abweichende Kopfhaltung eingenommen hatte (vg1. Figur 2c).

## Patentansprüche

1. Verfahren zur Zentrierdatenermittlung, bei dem
a. eine tatsächliche momentane Kopfhaltung eines Probanden bestimmt wird und mittels eines Videozentriergeräts konventionell Zentrierdaten ermittelt werden,
b. eine Anordnung einer Brillenfassung (202) relativ zur bestimmten tatsächlichen momentanen Kopfhaltung ermittelt wird, wobei
c. eine habituelle Kopfhaltung bestimmt wird, und
d. die bestimmte tatsächliche momentane Kopfhaltung mit Hilfe der bestimmten habituellen Kopfhaltung korrigiert wird,
**dadurch gekennzeichnet, dass** die habituelle Kopfhaltung mit einem automatisierten Verfahren mit folgenden Schritten bestimmt wird:
e. Aufzeichnung einer Kopfhaltung mittels eines am Kopf des Probanden angebrachten Positionsaufzeichnungsgeräts und/oder eines Bildaufzeichnungsgeräts (110) über eine Zeitspanne,
f. Ermittlung der habituellen Kopfhaltung aus der Aufzeichnung.

2. Verfahren nach Anspruch 1, **dadurch gekennzeichnet, dass** die Zeitspanne vorgegeben wird.

3. Verfahren nach einem der vorangegangenen Ansprüche, **dadurch gekennzeichnet, dass** als habituelle Kopfhaltung eine Kopfhaltung gewählt wird, in der der Proband seinen Kopf bei einer vorgegebenen Sehaufgabe mit einer Wahrscheinlichkeit von über 20 % oder von über 50 % hält.

4. Verfahren nach einem der vorangegangenen Ansprüche, **dadurch gekennzeichnet, dass** als habituelle Kopfhaltung die aufgezeichnete Haltung gewählt wird, in der der Proband den Kopf bei einer vorgegebenen Sehaufgabe mit der größten Wahrscheinlichkeit hält.

5. Verfahren nach einem der vorangegangenen Ansprüche, **dadurch gekennzeichnet, dass** als habituelle Kopfhaltung eine der aufgezeichneten Haltungen gewählt wird, in der der Proband den Kopf beim Blick in die Nähe und/oder beim Blick in die Ferne hält.

6. Verfahren nach einem der vorangegangenen Ansprüche, **dadurch gekennzeichnet, dass** als habituelle Kopfhaltung eine der aufgezeichneten Haltungen gewählt wird, während der der Proband steht oder sitzt.

7. Verfahren nach einem der vorangegangenen Ansprüche, **dadurch gekennzeichnet, dass** dem Probanden Alltagssituationen und/oder alltägliche Sehaufgaben dargeboten werden.

8. Vorrichtung zur Zentrierdatenermittlung mit
a. einer Kopfhaltungsbestimmungseinrichtung, die dazu eingerichtet ist, eine tatsächliche momentane Kopfhaltung eines Probanden zu bestimmen,
b. einer Ermittlungseinrichtung, die dazu eingerichtet ist, eine Anordnung einer Brillenfassung relativ zur bestimmten tatsächlichen momentanen Kopfhaltung zu ermitteln,
c. einer Vorrichtung eingerichtet zur Bestimmung einer habituellen Kopfhaltung, und
d. einer Korrektureinrichtung eingerichtet zur Korrektur der bestimmten tatsächlichen momentanen Kopfhaltung mit Hilfe der bestimmten habituellen Kopfhaltung,
**dadurch gekennzeichnet, dass** die Vorrichtung (100) zur automatisierten Bestimmung der habituellen Kopfhaltung ausgebildet ist und aufweist:
e. eine Aufzeichnungseinrichtung (110) eingerichtet zur Aufzeichnung einer Kopfhaltung über eine Zeitspanne, wobei die Aufzeichnungseinrichtung ein am Kopf des Probanden anbringbares Positionsaufzeichnungsgerät und/oder ein Bildaufzeichnungsgerät (112) umfasst;
f. eine Ermittlungseinrichtung (114) eingerichtet zur Ermittlung der habituellen Kopfhaltung aus der Aufzeichnung der Aufzeichnungseinrichtung (110).

9. Vorrichtung nach Anspruch 8, **dadurch gekennzeichnet, dass** die Ermittlungseinrichtung einen Computer (114) umfasst.

10. Computerprogramm mit Programmcode zur Durchführung des Verfahrens nach einem der vorangegangen Ansprüche 1 bis 7, wenn das Programm in einem Computer (114) der Ermittlungseinrichtung einer Vorrichtung nach einem der Ansprüche 8 oder 9 ausgeführt wird.

## Claims

1. Method for determining centring data, in which
a. an actual instantaneous head posture of a subject is determined and centring data are determined conventionally by means of a video centring device,
b. an arrangement of a spectacle frame (202) relative to the actual instantaneous head posture determined is determined, wherein
c. a habitual head posture is determined, and
d. the actual instantaneous head posture determined is corrected with the aid of the habitual head posture determined,
**characterized in that** the habitual head posture is determined by an automated method having the following steps:
e. recording a head posture over a period of time by means of a position recording device fitted to the subject's head and/or an image recording device (110),
f. determining the habitual head posture from the recording.

2. Method according to Claim 1, **characterized in that** the period of time is predefined.

3. Method according to any of the preceding claims, **characterized in that** the habitual head posture chosen is a head posture in which the subject keeps their head during a predefined vision task with a probability of over 20% or of over 50%.

4. Method according to any of the preceding claims, **characterized in that** the habitual head posture chosen is the recorded posture in which the subject keeps their head during a predefined vision task with the highest probability.

5. Method according to any of the preceding claims, **characterized in that** the habitual head posture chosen is one of the recorded postures in which the subject keeps their head during near-range viewing and/or during far-range viewing.

6. Method according to any of the preceding claims, **characterized in that** the habitual head posture chosen is one of the recorded postures during which the subject is standing or sitting.

7. Method according to any of the preceding claims, **characterized in that** the subject is presented with everyday situations and/or everyday vision tasks.

8. Apparatus for determining centring data, comprising
a. a head posture determining device configured to determine an actual instantaneous head posture of a subject,
b. a determining device configured to determine an arrangement of a spectacle frame relative to the actual instantaneous head posture determined,
c. an apparatus configured to determine a habitual head posture, and
d. a correction device configured to correct the actual instantaneous head posture determined with the aid of the habitual head posture determined,
**characterized in that** the apparatus (100) is designed to determine the habitual head posture in an automated manner and comprises:
e. a recording device (110) configured to record a head posture over a period of time, wherein the recording device comprises a position recording device that can be fitted to the subject's head and/or an image recording device (112);
f. a determining device (114) configured to determine the habitual head posture from the recording by the recording device (110).

9. Apparatus according to Claim 8, **characterized in that** the determining device comprises a computer (114).

10. Computer program comprising program code for carrying out the method according to any of the preceding Claims 1 to 7 if the program is executed in a computer (114) of the determining device of an apparatus according to either of Claims 8 and 9.

## Revendications

1. Procédé de détermination de données de centrage, dans lequel
a. un port de tête instantané réel d'un sujet est déterminé et des données de centrage conventionnelles sont déterminées au moyen d'un appareil de centrage vidéo,
b. un agencement d'une monture de lunettes (202) par rapport au port de tête instantané réel déterminé est déterminé,
c. un port de tête habituel étant déterminé et
d. le port de tête instantané réel déterminé étant corrigé à l'aide du port de tête habituel déterminé, **caractérisé en ce que** le port de tête habituel est déterminé par un procédé automatisé présentant les étapes suivantes :
e. enregistrement d'un port de tête au moyen d'un appareil d'enregistrement de position et/ou d'un appareil d'enregistrement d'images (110) fixé à la tête du sujet sur une période de temps,
f. détermination du port de tête habituel à partir de l'enregistrement.

2. Procédé selon la revendication 1, **caractérisé en ce que** la période de temps est spécifiée.

3. Procédé selon l'une des revendications précédentes, **caractérisé en ce qu'**on choisit, en tant que port de tête habituel, un port de tête dans lequel le sujet tient sa tête dans le cadre d'une tâche visuelle spécifiée avec une probabilité supérieure à 20% ou supérieure à 50%.

4. Procédé selon l'une des revendications précédentes, **caractérisé en ce qu'**on choisit, en tant que port de tête habituel, le port enregistré dans lequel le sujet tient sa tête dans le cadre d'une tâche visuelle spécifiée avec la plus grande probabilité.

5. Procédé selon l'une des revendications précédentes, **caractérisé en ce qu'**on choisit, en tant que port de tête habituel, l'un des ports enregistrés dans lequel le sujet tient la tête lorsqu'il regarde de près et/ou lorsqu'il regarde au loin.

6. Procédé selon l'une des revendications précédentes, **caractérisé en ce qu'**on choisit, en tant que port de tête habituel, l'un des ports enregistrés pendant que le sujet se tient debout ou assis.

7. Procédé selon l'une des revendications précédentes, **caractérisé en ce que** des situations quotidiennes et/ou des tâches visuelles quotidiennes sont présentées au sujet.

8. Dispositif pour la détermination de données de centrage, présentant
a. un système de détermination de port de tête conçu pour déterminer un port de tête instantané réel d'un sujet,
b. un système de détermination conçu pour déterminer un agencement d'une monture de lunettes par rapport au port de tête instantané réel déterminé,
c. un dispositif conçu pour déterminer un port de tête habituel et
d. un système de correction conçu pour corriger le port de tête instantané réel déterminé à l'aide du port de tête habituel déterminé,
**caractérisé en ce que** le dispositif (100) de détermination automatisée du port de tête habituel est réalisé et présente :
e. un système d'enregistrement (110) conçu pour enregistrer un port de tête sur une période de temps, le système d'enregistrement comprenant un appareil d'enregistrement de position et/ou un appareil d'enregistrement d'images (112) qui peut être fixé à la tête du sujet ;
f. un système de détermination (114) conçu pour déterminer le port de tête habituel à partir de l'enregistrement du système d'enregistrement (110).

9. Dispositif selon la revendication 8, caractérisé en ce le système de détermination comprend un ordinateur (114).

10. Programme informatique pourvu d'un code de programme destiné à mettre en œuvre le procédé selon l'une des revendications 1 à 7, lorsque le programme est exécuté dans un ordinateur (114) du système de détermination d'un dispositif selon l'une des revendications 8 ou 9.
